(19) Europäisches Patentamt
European Patent Office
Office européen des brevets

(11) Veröffentlichungsnummer: **0 389 417 A1**

(12) **EUROPÄISCHE PATENTANMELDUNG**

(21) Anmeldenummer: **90810176.9**

(22) Anmeldetag: **07.03.90**

(51) Int. Cl.[5]: **C07C 303/06, C07C 309/46, C07C 309/48, C07C 309/49, C09B 29/036**

(30) Priorität: **16.03.89 CH 979/89**

(43) Veröffentlichungstag der Anmeldung: **26.09.90 Patentblatt 90/39**

(84) Benannte Vertragsstaaten: **BE CH DE ES FR GB IT LI**

(71) Anmelder: **CIBA-GEIGY AG**
**Klybeckstrasse 141**
**CH-4002 Basel(CH)**

(72) Erfinder: **Herzig, Paul**
**Ackerstrasse 52**
**CH-4057 Basel(CH)**

(54) **Verfahren zur Herstellung von Aminobenzolsulfonsäuren.**

(57) Beschrieben wird ein Verfahren zur Herstellung von Verbindungen der Formel

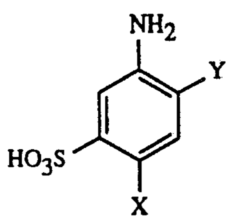

(1),

worin die Symbole, die im Anspruch 1 angegebene Bedeutung haben, welches dadurch gekennzeichnet ist, dass man eine Verbindung der Formel

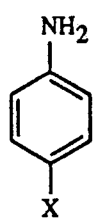

(2),

in Schwefelsäure löst, diese Lösung zu Oleum gibt und bei einer Temperatur von 10 bis 80 °C bis zur vollständigen Einführung einer Sulfogruppe reagieren lässt, und gegebenenfalls anschliessend das Reaktionsgemisch einer Weiterreaktion bei 100 bis 200 °C zur Einführung einer zweiten Sulfogruppe unterwirft.

Die nach dem Verfahren erhältlichen Verbindungen der Formel (1) stellen wertvolle Zwischenprodukte für die Herstellung von Farbstoffen dar und sind insbesondere geeignet als Diazokomponenten für die Herstellung von Azofarbstoffen.

EP 0 389 417 A1

# Verfahren zur Herstellung von Aminobenzolsulfonsäuren

Die vorliegende Erfindung betrifft ein Verfahren zur Herstellung von gegebenenfalls weitersubstituierten Aminobenzolmono- und -disulfonsäuren sowie die Verwendung der nach dem Verfahren erhältlichen Aminobenzolsulfonsäuren zur Herstellung von Farbstoffen.

Es ist bereits aus J. Chem. Soc. of Japan 1975, Seiten 1070-1075 ein Verfahren zur Herstellung von 4-Methylanilin-3-sulfonsäure bekannt, worin p-Toluidin in die 7fache Gewichtsmenge an 20%igem Oleum ohne zusätzliche Lösungs- oder Verdünnungsmittel eingetragen wird. Die nach dem Verfahren erhältliche 4-Methylanilin-3-sulfonsäure ist jedoch nur sehr unrein und weist einen beträchtlichen Anteil an unerwünschter 4-Methylanilin-2-sulfonsäure auf, der anschliessend umständlich entfernt werden muss. Zur Vermeidung solcher Isomerengemische wird in der EP-A 0 149 460 vorgeschlagen, umgekehrt das Oleum zur schwefelsauren Lösung von p-Toluidin zu tropfen. Die diesbezügliche Umsetzung verläuft jedoch mit Eigenexothermie und verlangt aufwendige sicherheitstechnische Vorkehrungen, z.B. eine genaue Temperatursteuerung, um sie unter Kontrolle zu halten; zudem müssen die Ansätze hinreichend Klein gewählt werden. Folglich lässt sich 4-Methylanilin-3-sulfonsäure nach dem aus der EP-A 0 149 460 bekannten Verfahren nur mit geringer Raum-Zeit-Ausbeute und unter grossem apparativen Aufwand herstellen.

Es besteht daher weiterhin das Bedürfnis nach einem einfach und mit grosser Raum-Zeit-Ausbeute auszuführenden Verfahren, nach welchem substituierte Aminobenzolmono- und disulfonsäuren mit höchster Selektivität hergestellt werden können; dieses wird mit der vorliegenden Erfindung zur Verfügung gestellt.

Gegenstand der Erfindung ist ein Verfahren zur Herstellung von Verbindungen der Formel

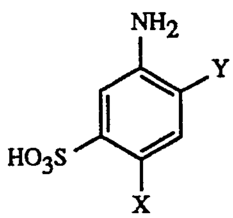

(1),

worin X Halogen, Hydroxy, gegebenenfalls substituiertes $C_1$-$C_4$-Alkyl oder gegebenenfalls substituiertes $C_1$-$C_4$-Alkoxy bedeutet und Y Wasserstoff oder Sulfo ist, dadurch gekennzeichnet, dass man eine Verbindung der Formel

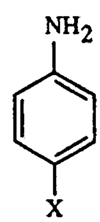

(2),

worin X die angegebene Bedeutung hat,
in 1,5 bis 10 Mol Schwefelsäure pro Mol Verbindung der Formel (2) löst, diese Lösung zu so viel Oleum gibt, dass pro Mol Verbindung der Formel (2) 2 bis 4 Mol freies $SO_3$ anwesend sind, und bei einer Temperatur von 10 bis 80 °C bis zur vollständigen Einführung einer Sulfogruppe reagieren lässt, und gegebenenfalls anschliessend das Reaktionsgemisch einer Weiterreaktion bei einer Temperatur von 100 bis 200 °C zur Einführung einer zweiten Sulfogruppe unterwirft.

Bei X als Halogen in den Verbindungen der Formeln (1) und (2) handelt es sich z.B. um Fluor, Brom oder vorzugsweise um Chlor.

Steht X für einen $C_1$-$C_4$-Alkylrest, so handelt es sich hierbei im weiteren generell um einen Methyl-, Ethyl-, n- oder iso-Propyl- oder n-, iso-, sec.- oder tert.-Butylrest; der Alkylrest X kann seinerseits substituiert sein, z.B. durch Halogen, insbesondere Chlor; Cyano; $C_1$-$C_4$-Alkoxy, worunter generell Methoxy, Ethoxy, n- oder iso-Propoxy oder n-, iso-, sec.- oder tert.-Butoxy zu verstehen ist; $C_1$-$C_4$-Alkoxycarbonyl, z.B. Methoxycarbonyl oder Ethoxycarbonyl; Carboxy; Sulfamoyl; Sulfo; oder Sulfato.

X als Alkylrest steht vorzugsweise für einen unsubstituierten $C_1$-$C_2$-Alkylrest und besonders bevorzugt für den Methylrest.

Bedeutet X einen $C_1$-$C_4$-Alkoxyrest, so kann dieser gegebenenfalls einen oder mehrere der zuvor für den Alkylrest X genannten Substituenten aufweisen; X steht als Alkoxy vorzugsweise für unsubstituiertes $C_1$-$C_2$-Alkoxy und besonders bevorzugt für Methoxy.

Y in den Verbindungen der Formel (1) steht vorzugsweise für Sulfo.

Das erfindungsgemässe Verfahren ist insbesondere geeignet zur Herstellung von Verbindungen der zuvor angegebenen Formel (1), worin X Chlor, Methyl oder Ethyl und Y Sulfo ist.

Eine besonders bevorzugte Ausführungsform des erfindungsgemässen Verfahrens betrifft die Herstellung von 4-Methylanilin-2,5-disulfonsäure.

Die Verbindung der Formel (2) wird erfindungsgemäss in schwefelsaurer Lösung eingesetzt und hierbei wird 1,5 bis 10 Mol, vorzugsweise 1,5 bis 5, und insbesondere bevorzugt 1,5 bis 3 Mol $H_2SO_4$ pro Mol Verbindung der Formel (2) verwendet. Im allgemeinen wird hierzu eine 100%ige $H_2SO_4$ (sogenanntes Monohydrat) eingesetzt. Es ist jedoch auch möglich, Schwefelsäuren mit einem geringen Wassergehalt, z.B. bis zu 8, bevorzugt bis zu 5% $H_2O$, bezogen auf die Gesamtmenge der Säure, zu verwenden.

Dies bedingt jedoch gegebenenfalls, dass die Menge des für die Sulfierung im Reaktionsgefäss vorzulegenden Oleums entsprechend dem Wassergehalt der $H_2SO_4$ erhöht werden muss, da ja dieses Wasser einen Teil des im Oleum vorhandenen $SO_3$ verbraucht.

Die Zugabe der Verbindung der Formel (2) zur Schwefelsäure kann in fester Form, z.B. als Pulver, oder vorzugsweise als Schmelze erfolgen, wobei die verwendete Temperatur innerhalb weiter Grenzen variieren kann; als vorteilhaft hat sich eine Temperatur von z.B. 40 bis 100 °C, vorzugsweise 50 bis 85 °C, herausgestellt. Es erweist sich darüberhinaus oft als günstig, nach erfolgter Zugabe der Verbindung der Formel (2) zur Schwefelsäure noch einige Minuten bei der angegebenen Temperatur weiterzurühren, um eine homogene, klare Lösung zu erhalten.

Bei dem verwendeten Oleum kann es sich um jedes übliche Oleum handeln. Das Oleum weist zweckmässigerweise einen $SO_3$-Gehalt von z.B. 20 bis 70 Gew.-% und vorzugsweise von 60 bis 66 Gew.-% auf. Erfindungsgemäss legt man so viel Oleum vor, dass pro Mol zuzugebender Verbindung der Formel (2) 2 bis 4 Mol und vorzugsweise 2 bis 3 Mol $SO_3$ anwesend sind.

Die Temperatur des Reaktionsgemisches wird während der Zugabe der schwefelsauren Lösung der Verbindung der Formel (2) zum Oleum zweckmässigerweise mittels eines Eisbades z.B. bei 10 bis 70 °C und vorzugsweise bei 15 bis 55 °C gehalten; gegebenenfalls kann die Temperatur auch während der Zugabe innerhalb der angegebenen Grenzen variiert werden.

Die für die Zugabe der schwefelsauren Lösung der Verbindung der Formel (2) zum Oleum benötigte Zeit kann innerhalb weiter Grenzen variieren, wobei sich eine Zeit von ca. 20 Minuten bis 5 Stunden, vorzugsweise 30 Minuten bis 2,5 Stunden als praktikabel erwiesen hat.

Nachdem die Zugabe der Verbindung der Formel (2) zum Oleum vollständig ist, schliesst sich vorzugsweise eine Nachreaktion bei z.B. 10 bis 80 °C, vorzugsweise 15 bis 75 °C, an, um gegebenenfalls noch nicht umgesetzte Verbindung der Formel (2) zur Reaktion zu bringen. Das Vorhandensein von nicht umgesetztem Ausgangsprodukt kann dabei z.B. mittels Dünnschichtchromatographie an einer Probe des Reaktionsgemisches festgestellt werden. Man verwendet z.B. eine Nachreaktionszeit von 10 Minuten bis 3 Stunden. Im allgemeinen ist jedoch die Umsetzung nach etwa 15 bis 30 Minuten vollständig und dementsprechend keine Ausgangsverbindung der Formel (2) mehr im Reaktionsgemisch nachweisbar.

Die wie oben geschildert erhaltene Monosulfo-Verbindung der Formel (1) [Y = Wasserstoff] kann wie weiter unten geschildert aufgearbeitet werden oder vorzugsweise anschliessend direkt in die Disulfo-Verbindung der Formel (1) [Y = Sulfo] überführt werden, indem man das Reaktionsgemisch auf eine Temperatur von 100 bis 200 °C, vorzugsweise 130 bis 160 °C, erhitzt und bei dieser Temperatur weiterreagieren lässt; als vorteilhaft erweist sich hierbei eine Reaktionszeit von z.B. 1 bis 10 Stunden, vorzugsweise 1,5 bis 5 Stunden und besonders bevorzugt 2,5 bis 4 Stunden.

Eine besonders bevorzugte Ausführungsform der vorliegenden Erfindung betrifft ein Verfahren zur Herstellung von 4-Methylanilin-2,5-disulfonsäure, worin man 4-Methylanilin in 1,5 bis 3 Mol Schwefelsäure pro Mol 4-Methylanilin löst, diese Lösung zu so viel 60 bis 66 %-igem Oleum gibt, dass pro Mol 4-Methylanilin 2 bis 3 Mol freies $SO_3$ anwesend sind, und bei einer Temperatur von 10 bis 80 °C 10 Minuten bis 3 Stunden nachreagieren lässt, anschliessend das Reaktionsgemisch auf 130 bis 160 °C erhitzt und 2,5 bis 4 Stunden lang bei dieser Temperatur weiterreagieren lässt.

Die Aufarbeitung des Reaktionsgemisches erfolgt jeweils in an sich bekannter Weise, z.B. indem man Wasser zugibt bzw. das Reaktionsgemisch in Wasser einträgt. Wasser kann hierbei z.B. als solches, als Eis/Wassergemisch oder in Form von zerkleinertem Eis eingesetzt werden. Nach dem Versetzen des Reaktionsgemisches mit Wasser fällt die Verbindung der Formel (1) als freie Säure aus und kann durch übliche Methoden, wie Filtration oder Abzentrifugieren, gewonnen werden.

Es ist darüberhinaus auch möglich, und bevorzugt, die Verbindung der Formel (1) in Form des Mono-

bzw. Dialkalisalzes zu isolieren, indem man das Reaktionsgemisch zunächst wiederum in Wasser bzw. Eis einträgt, daraufhin eine Alkalimetallbase, z.B. Lithium-, Natrium- oder Kaliumhydroxid oder -carbonat oder ein Alkalimetallsalz wie z.B. Natriumsulfat oder Kaliumsulfat, im Überschuss zusetzt und das ausgefallene Alkalisalz der Verbindung der Formel (1) abfiltriert oder abzentrifugiert.

Das erfindungsgemässe Verfahren bietet z.B. gegenüber dem aus der EP-A 0 149 460 bekannten grosse sicherheitstechnische Vorteile. Während bei letzterem erst eine bestimmte Menge Oleum zur Verbindung der Formel (2) zugetropft werden muss, um die Reaktion überhaupt in Gang zu setzen, und die Umsetzung dann aufgrund der gesamten anwesenden Edukt-Menge sehr stürmisch und mit Eigenexothermie verläuft, reagiert beim erfindungsgemässen Verfahren die verdünnt in das Oleum eingetragene Verbindung der Formel (2) spontan weg; d.h., die Exothermie kann über die Zugabegeschwindigkeit der Verbindung der Formel (2) gesteuert und das Verfahren dementsprechend mit grosser Raum-Zeit-Ausbeute ausgeführt werden.

Es muss andererseits aufgrund der spontanen Reaktion der Verbindung der Formel (2) mit dem Oleum und angesichts der Offenbarung des J. Chem. Soc. of Japan 1975, Seiten 1070-1075 als überraschend bezeichnet werden, dass die Verbindungen der Formel (1) nach dem erfindungsgemässen Verfahren mit überaus hoher Reinheit erhalten werden:

So fallen die Monosulfo-Verbindungen der Formel (1) [Y = Wasserstoff] praktisch frei von Ausgangsprodukt der Formel (2) und störender isomerer Verbindung der Formel

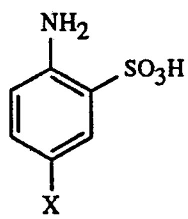

(1a),

worin X die zuvor angegebene Bedeutung hat, an.

Insbesondere überraschend werden auch die Disulfo-Verbindungen der Formel (1) [Y = Sulfo] mit einer Reinheit von > 97 %, vorzugsweise ≧ 99 % erhalten; im Reaktionsprodukt ist dabei praktisch kein störendes Isomer der Formel

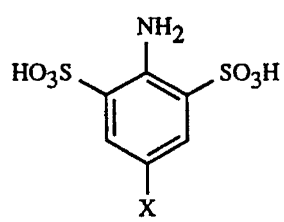

(1b),

worin X die zuvor angegebene Bedeutung hat, mittels Hochdruck-Flüssigkeitschromatographie (HPLC) mehr nachweisbar.

Dementsprechend kann die Isolierung der Verbindungen der Formel (1) ohne zusätzliche Reinigungsstufe, wie Waschen oder Umkristallisieren, erfolgen.

Die Verbindungen der Formel (1) sind zum Teil neu. Ein weiterer Gegenstand der vorliegenden Erfindung betrifft dementsprechend Verbindungen der zuvor angegebenen Formel (1), worin Y Sulfo und X Fluor, Chlor, Brom oder $C_2$-$C_4$-Alkyl ist.

Die nach dem erfindungsgemässen Verfahren erhältlichen Verbindungen der Formel (1) stellen wertvolle Zwischenprodukte z.B. für die Herstellung von Farbstoffen dar; sie sind insbesondere geeignet als Diazokomponente für die Herstellung von Azofarbstoffen.

Eine besonders bevorzugte Verwendung der nach dem erfindungsgemässen Verfahren erhältlichen 4-Methylanilin-2,5-disulfonsäure betrifft deren Verwendung als Diazokomponente für die Herstellung von faserreaktiven Azofarbstoffen und insbesondere für die Herstellung von Farbstoffen der Formel

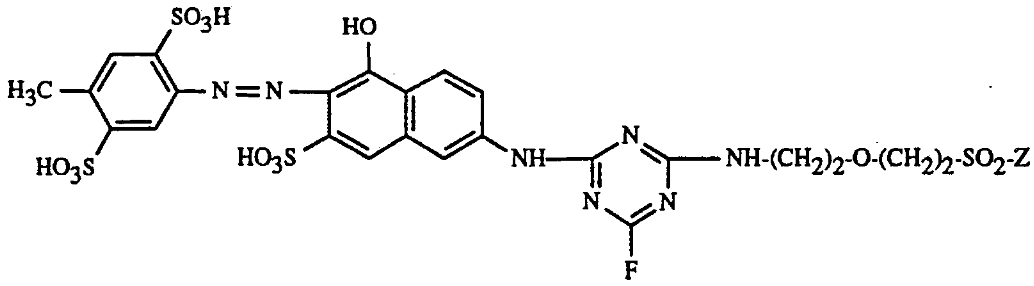

(3),

worin Z einen Rest $-CH_2-CH_2-A$ oder vorzugsweise Vinyl bedeutet und A -Cl, $-OSO_3H$, $-SSO_3H$, $-OCOCH_3$ oder $-OCO-C_6H_5$ ist.

Die nachfolgenden Beispiele dienen der Erläuterung der Erfindung, ohne sie darauf zu beschränken. Teile bedeuten Gewichtsteile und Prozente Gewichtsprozente.

Beispiel 1:

107 Teile geschmolzenes p-Toluidin werden bei 50 bis 55° C in 240 Teile 100%iger Schwefelsäure eingetropft. Man rührt 30 Minuten nach und lässt dann die erhaltene schwefelsaure Lösung innerhalb von 2 Stunden in 320 Teile 66%iges Oleum einlaufen, wobei die Temperatur mittels Eiskühlung bei 15-20° C gehalten wird. Nach weiteren 15 Minuten bei 20° C ist mittels HPLC kein p-Toluidin mehr im Reaktionsgemisch nachweisbar.

Das Reaktionsgemisch wird anschliessend auf 150° C erwärmt und 3 Stunden bei dieser Temperatur gerührt. Man kühlt dann auf 50° C ab, trägt die Reaktionsmasse auf 900 Teile Eis aus und tropft zur entstehenden klaren Lösung 370 Teile 50 %ige Kaliumhydroxidlösung bei 20 bis 30° C zu. Anschliessend wird auf 5° C abgekühlt und das ausgefallene Produkt abfiltriert. Es ergeben sich 411 Teile einer feuchten 4-Methylanilin-2,5-disulfonsäure mit einem Gehalt von 56 %, was einer Ausbeute von 86 %, bezogen auf eingesetztes p-Toluidin, entspricht. Das Produkt weist eine 99%ige Reinheit auf.

Beispiel 2:

Verfährt man wie in Beispiel 1 beschrieben, trägt jedoch die Sulfiermasse auf 1000 Teile Eiswasser aus und salzt die Lösung mit 290 Teilen Kaliumsulfat aus, so erhält man nach der Filtration 414 Teile einer feuchten 4-Methylanilin-2,5-disulfonsäure mit einem Gehalt von 60 %, was einer Ausbeute von 93 % bezogen auf eingesetztes p-Toluidin entspricht. Das Produkt weist eine 99,5%ige Reinheit auf.

Beispiel 3:

In 238 Teile 100 %iger Schwefelsäure werden bei einer Temperatur von max. 85° C 128 Teile p-Chloranilin in Portionen eingetragen. Danach wird 15 Minuten bei 65° C nachgerührt, bis eine klare Lösung entsteht. Diese Schwefelsäure-Lösung wird innerhalb von 30 Minuten zu 420 Teilen 66 %igem Oleum zulaufen gelassen. Die Temperatur hält man dabei mit einem Eisbad bei 65-70° C. Zur Vervollständigung der Reaktion zu 4-Chloranilin-3-sulfonsäure wird noch 2 Stunden bei 70° C nachgerührt. Dann erwärmt man auf 160° C und rührt 3 Stunden bei dieser Temperatur. Danach kühlt man auf 50° C ab. Die Sulfierungsmasse wird auf 1100 Teile Eis/Wasser ausgetragen und die entstehende klare Lösung mit 400 Teilen Kaliumhydroxid-Lösung 50%ig teilneutralisiert. Das Mono-Kaliumsalz der 4-Chloranilin-2,5-disulfonsäure fällt langsam aus, wird abgesaugt und im Vakuum getrocknet.

Man erhält 260 Teile 4-Chloranilin-2,5-disulfonsäure mit einem Gehalt von 62,0 %. Dies entspricht einer Ausbeute von 56 %, bezogen auf das eingesetzte p-Chloranilin. Die HPLC-Reinheit beträgt > 97%.

Beispiel 4:

In 183 Teile 100%iger Schwefelsäure werden 121 Teile 4-Ethylanilin bei einer Temperatur von 75-85° C eingetropft. Nach weiteren 15 Minuten Nachrühren bei 80° C entsteht eine klare Lösung. Diese Schwefelsäure-Lösung wird innerhalb von 40 Minuten zu 320 Teilen 66%igem Oleum zugetropft. Die Temperatur hält man mittels Eisbad bei 60-65° C. Nach 15 Minuten Nachrühren bei 65° C ist kein 4-Ethylanilin mehr nachweisbar. Es wird noch 4 Stunden bei 150° C gerührt, dann die Sulfierungsmasse auf 80° C abgekühlt und auf 900 Teile Eis/Wasser ausgetragen. Durch Eintropfen von 370 Teilen 50%-iger KOH-Lösung wird das Mono-Kaliumsalz der 4-Ethylanilin-2,5-disulfonsäure ausgefällt und durch Filtration isoliert.

Man erhält 350 Teile feuchte 4-Ethylanilin-2,5-disulfonsäure mit einem Gehalt von 53,0 %. Dies entspricht einer Ausbeute von 66 %, bezogen auf eingesetztes 4-Ethylanilin. Die HPLC-Reinheit beträgt > 99%.

Beispiel 5:

In 183 Teile 100 %iger Schwefelsäure werden innerhalb von 30 Minuten 107 Teile geschmolzenes p-Toluidin eingetropft und dabei die Temperatur bei 75-80° C gehalten. Nach weiteren 15 Minuten Nachrühren ist das p-Toluidin vollständig gelöst. Diese Schwefelsäure-Lösung wird innerhalb von 30 Minuten zu 260 Teilen 66%igem Oleum zulaufen gelassen. Die Temperatur hält man mit einem Eisbad bei 40-50° C. Nach weiteren 30 Minuten Nachrühren bei 45-50° C wird mittels HPLC kein Edukt mehr nachweisbar. Der Ansatz wird auf 1000 ml Eiswasser ausgetragen, die ausgefallene 4-Methylanilin-3-sulfonsäure abgesaugt und mit 500 Teilen Eiswasser gewaschen. Man erhält 248 Teile feuchte 4-Methylanilin-3-sulfonsäure mit einem Gehalt von 70,0 %; dies entspricht einer Ausbeute von 93 %, bezogen auf das eingesetzte p-Toluidin. Mittels HPLC ist kein p-Toluidin und keine 4-Methylanilin-2-sulfonsäure mehr nachweisbar (< 0,1 %).

Beispiel 6:

In 240 Teile 100%iger Schwefelsäure werden innerhalb von 30 Minuten 123 Teile geschmolzenes p-Anisidin eingetropft und dabei die Temperatur bei 55-60° C gehalten. Nach weiteren 15 Minuten ist das p-Anisidin vollständig gelöst. Diese Schwefelsäure-Lösung wird innerhalb von 30 Minuten zu 242 Teilen 66%igem Oleum zulaufen gelassen. Die Temperatur hält man mit einem Eisbad bei 30-40° C. Nach weiteren 30 Minuten Nachrühren bei 40° C ist mittels HPLC kein Edukt mehr nachweisbar. Der Ansatz wird auf 1000 Teile Eiswasser ausgetragen, die ausgefallene 4-Methoxyanilin-3-sulfonsäure abgesaugt und mit 500 Teilen Eiswasser gewaschen. Nach dem Trocknen erhält man 179 Teile 4-Methoxyanilin-3-sulfonsäure mit einem Gehalt von 97,0 %; dies entspricht einer Ausbeute von 85 %, bezogen auf das eingesetzte p-Anisidin. Mittels HPLC ist kein p-Anisidin und keine 4-Methoxyanilin-2-sulfonsäure mehr nachweisbar (< 0,1%).

## Ansprüche

1. Verfahren zur Herstellung von Verbindungen der Formel

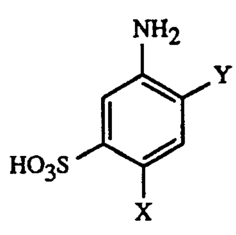

(1),

worin X Halogen, Hydroxy, gegebenenfalls substituiertes $C_1$-$C_4$-Alkyl oder gegebenenfalls substituiertes $C_1$-$C_4$-Alkoxy bedeutet und Y Wasserstoff oder Sulfo ist, dadurch gekennzeichnet, dass man eine Verbindung der Formel

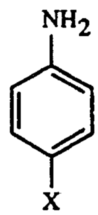

(2),

worin X die angegebene Bedeutung hat, in 1,5 bis 10 Mol Schwefelsäure pro Mol Verbindung der Formel (2) löst, diese Lösung zu so viel Oleum gibt, dass pro Mol Verbindung der Formel (2) 2 bis 4 Mol freies $SO_3$ anwesend sind, und bei einer Temperatur von 10 bis 80 °C bis zur vollständigen Einführung einer Sulfogruppe reagieren lässt, und gegebenenfalls anschliessend das Reaktionsgemisch einer Weiterreaktion bei einer Temperatur von 100 bis 200 °C zur Einführung einer zweiten Sulfogruppe unterwirft.

2. Verfahren gemäss Anspruch 1, dadurch gekennzeichnet, dass in den Verbindungen der Formel (1) X Chlor, Methyl oder Ethyl und Y Sulfo ist.

3. Verfahren gemäss Anspruch 1 oder 2 zur Herstellung von 4-Methylanilin-2,5-disulfonsäure.

4. Verfahren gemäss Anspruch 1 bis 3, dadurch gekennzeichnet, dass man 1,5 bis 5 Mol und vorzugsweise 1,5 bis 3 Mol Schwefelsäure pro Mol Verbindung der Formel (2) einsetzt.

5. Verfahren gemäss Anspruch 1 bis 4, dadurch gekennzeichnet, dass man ein Oleum mit einem $SO_3$-Gehalt von 60 bis 66 Gew.-% verwendet.

6. Verfahren gemäss Anspruch 1 bis 5, dadurch gekennzeichnet, dass man eine Weiterreaktion bei einer Temperatur von 130 bis 160 °C zur Einführung einer zweiten Sulfogruppe anschliesst.

7. Verfahren gemäss Anspruch 1 bis 6, dadurch gekennzeichnet, dass die Isolierung der Verbindungen der Formel (1) ohne zusätzliche Reinigungsstufe erfolgt.

8. Verfahren zur Herstellung von 4-Methylanilin-2,5-disulfonsäure, dadurch gekennzeichnet, dass man 4-Methylanilin in 1,5 bis 3 Mol Schwefelsäure pro Mol 4-Methylanilin löst, diese Lösung zu so viel 60 bis 66%igem Oleum gibt, dass pro Mol 4-Methylanilin 2 bis 3 Mol freies $SO_3$ anwesend sind, und bei einer Temperatur von 10 bis 80 °C 10 Minuten bis 3 Stunden nachreagieren lässt, anschliessend das Reaktionsgemisch auf 130 bis 160 °C erhitzt und 2,5 bis 4 Stunden lang bei dieser Temperatur weiterreagieren lässt.

9. Verfahren gemäss Anspruch 8, dadurch gekennzeichnet, dass die 4-Methylanilin-2,5-disulfonsäure mit einer Reinheit von > 97%, vorzugsweise ≧ 99%, anfällt.

10. Verwendung der nach dem Verfahren gemäss einem der Ansprüche 1 bis 9 erhältlichen Verbindungen der Formel (1) zur Herstellung von Farbstoffen.

11. Verwendung gemäss Anspruch 10 der Verbindungen der Formel (1) als Diazokomponente für die Herstellung von Azofarbstoffen.

12. Verwendung gemäss Anspruch 10 oder 11 von 4-Methylanilin-2,5-disulfonsäure als Diazokomponente zur Herstellung von faserreaktiven Azofarbstoffen, insbesondere zur Herstellung von Farbstoffen der Formel

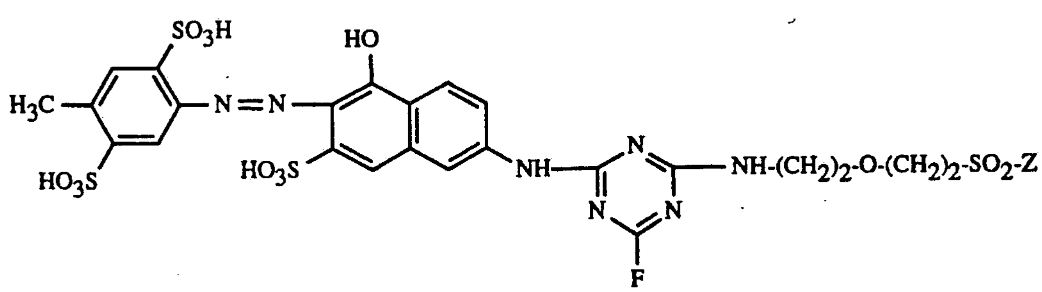

(3),

worin Z Vinyl oder einen Rest $-CH_2-CH_2A$ und A $-Cl$, $-OSO_3H$, $-SSO_3H$, $-OCOCH_3$ oder $-OCO-C_6H_5$ bedeutet.

13. Verbindungen der Formel (1) gemäss Anspruch 1, worin Y Sulfo und X Fluor, Chlor, Brom oder $C_2$-$C_4$-Alkyl ist.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 90 81 0176

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP-A-0 149 460 (BAYER)<br>* Beispiele, Ansprüche *<br>-- | 1-5 | C 07 C 303/06<br>C 07 C 309/46<br>C 07 C 309/48<br>C 07 C 309/49<br>C 09 B 29/036 |
| A | DE-A-1 817 986 (BAYER)<br>* Insgesamt *<br>-- | 1-12 | |
| X | CHEMICAL ABSTRACTS, Tenth Collective Index, Band 86-95, 1977-1981, Formulas $C_6H_5$-$C_8H_{24}N_4Zn$, Seite 1688F, $C_6H_6ClNO_6S_2$, 1,4-Benzenedisulfonic acid, 2-amino, 5-chloro, Columbus, Ohio, US;<br>* Zusammenfassung *<br>& JP-A-53 102 329 (SHOWA CHEMICAL INDUSTRIES)<br>& JP-A-52 078 235 (SHOWA CHEMICAL INDUSTRIES)<br>---- | 13 | |

RECHERCHIERTE SACHGEBIETE (Int. Cl.4)

C 07 C 303/00
C 07 C 309/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche: 8,9,13
Unvollständig recherchierte Patentansprüche: 1-7,10-12
Nicht recherchierte Patentansprüche:
Grund für die Beschränkung der Recherche:

Der in Anspruch 1 benutzte Ausdruck "gegebenenfalls substituiertes $C_1$-$C_4$ Alkyl oder gegebenenfalls substituiertes $C_1$-$C_4$ Alkoxy" definiert des Anmeldungsgegenstand unzureichend und erlaubt keine vollständige Recherche. (Art. 84 EPA)

| Recherchenort | Abschlußdatum der Recherche | Prüfer |
|---|---|---|
| DEN HAAG | 30-05-1990 | ZAROKOSTAS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument